(19) **Europäisches Patentamt / European Patent Office / Office européen des brevets**

(11) **EP 3 318 179 B1**

(12) # EUROPEAN PATENT SPECIFICATION

(45) Date of publication and mention of the grant of the patent:
**22.03.2023   Bulletin 2023/12**

(21) Application number: **15897267.9**

(22) Date of filing: **07.12.2015**

(51) International Patent Classification (IPC):
**A61B 5/0535** $^{(2021.01)}$    **A61B 5/11** $^{(2006.01)}$
**A61B 5/113** $^{(2006.01)}$    **A61B 5/00** $^{(2006.01)}$
**A61B 5/0205** $^{(2006.01)}$    **A61B 5/024** $^{(2006.01)}$
**A61B 5/08** $^{(2006.01)}$

(52) Cooperative Patent Classification (CPC):
**A61B 5/0205; A61B 5/02416; A61B 5/02438;
A61B 5/0535; A61B 5/0816; A61B 5/1128;
A61B 5/1135; A61B 5/6823; A61B 5/6898;
A61B 5/7246; A61B 5/725; A61B 5/743;**
A61B 5/7257

(86) International application number:
**PCT/KR2015/013303**

(87) International publication number:
**WO 2017/003049 (05.01.2017 Gazette 2017/01)**

(54) **METHOD FOR MEASURING RESPIRATION RATE AND HEART RATE USING DUAL CAMERA OF SMARTPHONE**

VERFAHREN ZUR MESSUNG DER ATEMFREQUENZ UND DER HERZFREQUENZ MIT EINER DOPPELKAMERA EINES SMARTPHONES

PROCÉDÉ POUR MESURER LA FRÉQUENCE RESPIRATOIRE ET LA FRÉQUENCE CARDIAQUE À L'AIDE DES DEUX CAMÉRAS D'UN TÉLÉPHONE INTELLIGENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **01.07.2015   KR 20150094242**
                  **03.12.2015   KR 20150171253**

(43) Date of publication of application:
**09.05.2018   Bulletin 2018/19**

(73) Proprietor: **Soonchunhyang University Industry Academy
Cooperation Foundation
Asan-si, Chungcheongnam-do 31358 (KR)**

(72) Inventors:
  • **NAM, Yun-Young
    Asan-si
    Chungcheongnam-do 31579 (KR)**

  • **KONG, Young-Sun
    Seoul 07648 (KR)**
  • **REYES, Bersain A.
    Coventry, Connecticut 06238 (US)**
  • **CHON, Ki H
    Storrs, CT 06269-3247 (US)**
  • **RELJIN, Natasa
    Coventry, Connecticut 06238 (US)**

(74) Representative: **Verscht, Thomas Kurt Albert
Josephsburgstrasse 88 A
81673 München (DE)**

(56) References cited:
**KR-A- 20150 016 903      US-A1- 2011 251 493
US-A1- 2013 215 244      US-A1- 2015 124 067**

## Description

### Technical Field

The present invention relates to a method for simultaneously measuring a respiration rate and a heart rate using dual cameras of a smartphone.

### Background Art

Recently, performances of portable devices, such as mobile phones, smartpads, and especially smartphones have been rapidly enhanced such that various functions that could not be realized before can now be performed. Particularly, recent smartphones contain various types of sensors, such as acceleration sensors, gyroscopes, touch sensors, temperature sensors, illumination sensors, gravity sensors, geomagnetic sensors, position sensors, etc., and high-performance cameras with innovative enhancements in hardware due to enhancements in computation speed. The hardware performance enhancement of the smartphones does not simply mean function enhancement, but it enables various applications to be received from an application server and to be installed in smartphones, whereby application fields of the smartphones are dramatically expanded. As one of the expanded application fields of smartphones, there is the medical field. A smartphone is likely to be carried by a user at all times due to the characteristics of such a portable terminal. Thus, smartphones are suitable for the medical field where the health condition of the user needs to be checked frequently. As enhanced functions of the smartphone, various medical applications are installed in the smartphone such that the smartphone can monitor the health condition of the user in various ways.

The most common method of measuring respiration is to use respiratory sounds with chest movements or auscultation. These methods are constrained by accuracy or irregularity.

In the case of electrocardiography, a sensor senses heart rate signals of a patient in an analog form and transmits the signals to a dedicated terminal through a USB cable, etc., and then the dedicated terminal converts the analog heart rate signal into digital heart rate signals using a micro control unit (MCU), an analog-to-digital converter (ADC), etc. to perform analysis.

However, a currently used electrocardiography device is problematic in that the MCU, etc. must be embedded in the dedicated terminal to convert the analog heart rate signals obtained through the heart rate sensor into the digital heart rate signals, and thus there are substantial errors in accuracy and manufacturing costs and sale prices are extremely high. Therefore, recently, in order to lower the manufacturing costs, an electrocardiography device with the MCU removed from the dedicated terminal has been developed. However, in this case, the heart rate sensor must contain the MCU to perform analog-to-digital conversion, and thus the existing problems still cannot be solved.

The background of the present invention is disclosed in Korean Patent Application Publication No. 10-2012-0066868 (25 June 2012).

From US 2015/124 067 A1 a handheld device having at least one illuminator for projecting source light and a video camera for capturing images of a region of interest of a subject being monitored for a desired physiological function is known.

From US 2011/251 493 A1 a method a system for measuring physiological parameters is known wherein the method includes capturing a sequence of images of a human face and identifying the location of the face in a frame of the video and establishing a region of interest including the face.

From KR 1015 016 903 A a method of measuring a pulse wave by using a mobile terminal such as a smart phone is known, wherein an oxygen saturation of a blood vessel varies according to pressures corresponding to systole and diastole of a heart, and absorption of light varies accordingly.

From US 2013/215 244 A1 a system and method for automatically removing undesirable periodic or random background noise from heart rate measurement signals obtained from a video camera, ambient illuminator and other unknown electromagnetic sources to improve the overall reliability of biomedical measurements is known.

### Disclosure

### Technical Problem

An object of the present invention is to provide a method for simultaneously measuring a respiration rate and a heart rate using dual cameras of a smartphone.

Also, the present invention is to enhance accuracy by simultaneously using respiration and PPG data.

Also, an object of the present invention is to provide a method continuously monitoring a heart rate and a respiration rate so as to prevent death from cardiac arrest.

## Technical Solution

According to an embodiment of the present invention, there is provided a method for measuring a respiration rate and a heart rate using dial cameras of a smartphone, the method including: simultaneously collecting, at a first step, a PPG signal of a human and visual data on chest and abdomen movements of the human using the dual cameras; converting, at a second step, the visual data to an RGBA signal; applying, at a third step, a bandpass filter such that a frequency only within a range in the RGBA signal passes; removing, at a fourth step, first and last predetermined portions of the RGBA signal to which the band-pass filter is applied at the third step; selecting, at a fifth step, a color channel having a highest standard deviation from the RGBA signal where the predetermined portions are removed at the fourth step; applying, at a sixth step, a spline filter to the color channel selected at the fifth step; computing, at a seventh step, power spectrum density using the result of the sixth step; and computing, at an eighth step, a heart rate and a respiration rate using the result of the seventh step.

## Advantageous Effects

According to the present invention, a respiration rate and a heart rate can be simultaneously measured with high accuracy using dual cameras of a smartphone.
Also, a heart rate and a respiration rate can be continuously monitored such that sudden death and death from cardiac arrest can be prevented.

## Description of Drawings

FIGS. 1a and 1b are views illustrating a screen for collecting data to simultaneously measure a respiration rate and a heart rate using dual cameras of a smartphone according to an embodiment of the present invention.
FIG. 2 is a flowchart illustrating a method for simultaneously measuring a respiration rate and a heart rate using dual cameras of a smartphone according to an embodiment of the present invention.
FIG. 3a is a graph illustrating a respiration rate for a chest movement measured using a front side camera of a smartphone.
FIG. 3b is a graph illustrating a respiration rate for an abdomen movement measured using a front side camera of a smartphone.
FIG. 4a is a Bland-Altman graph for comparing measurement values of a heart rate using a smartphone and ECG values.
FIG. 4b is a correlation graph for comparing measurement values of a heart rate using a smartphone and ECG values.
FIG. 5 is a view illustrating a respiration rate and a PPG signal of a human measured according to an embodiment of the present invention.

## Best Mode

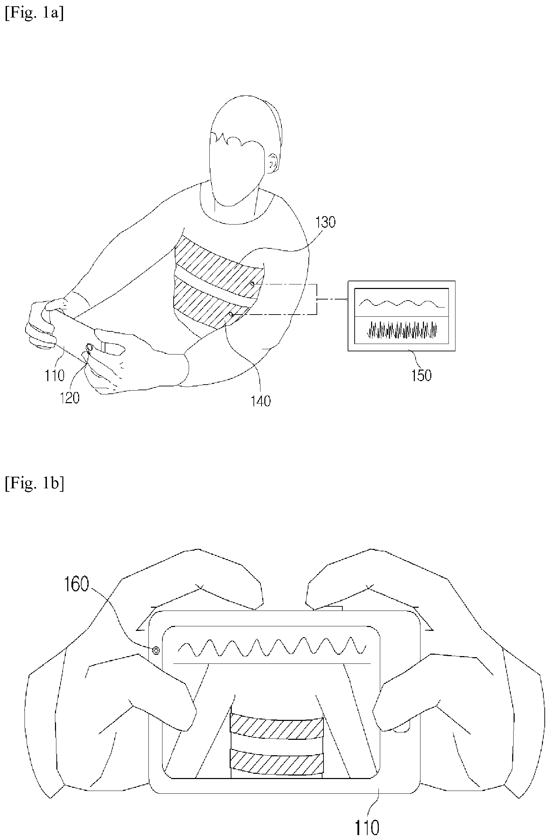

Hereinafter, the present invention will be described in detail with reference to the accompanying drawings.
As shown in FIGS. 1a and 1b, for a method for measuring a respiration rate and a heart rate using dual cameras of a smartphone according to an embodiment of the present invention, a smartphone 110, a chest impedance belt 130, an abdomen impedance belt 140, and a monitoring device 150 are provided.
First, a human wears impedance belts on the chest and abdomen to collect data. The chest and abdomen impedance belts 130 and 140 are intended to measure an actual respiration rate of a human, and are coupled to the monitoring device 150.
As shown in FIG. 1a, while a human is wearing the chest and abdomen impedance belts 130 and 140, a front side camera 160 of the smartphone 110 records chest and abdomen movements thereof and a rear side camera 120 of the smartphone 110 is in contact with the finger of the human for a preset time to measure a photoplethysmogram (PPG). Here, the measurement is performed with a turned on flash that is built in the rear side camera 120. As shown in FIG. 1b, the human may verify a video recording the chest and abdomen movements through the front side camera 160 of the smartphone 110, a PPG signal being measured, and the respiration rate for the chest and abdomen movements in real time. Also, in the present invention, for accuracy of the measurement of the respiration rate, the respiration rate of the human measured using the front side camera 160 is compared with the respiration rate measured by the impedance belt.
Video signals of the video recorded by the front side camera 160 are stored in a range of 20 to 25 Hz, and audio signals are stored at 44.100 Hz and 16 bits. Also, mono audio signals have noise removed by applying a band-pass filter within a range of 500 to 5000 Hz.

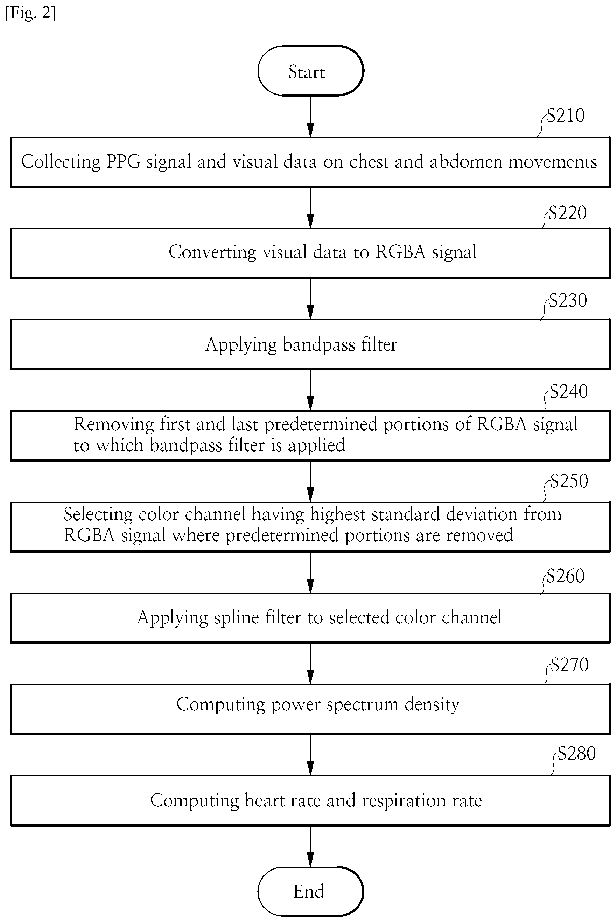

FIG. 2 is a flowchart illustrating detailed steps of a method for simultaneously measuring a respiration rate and a heart rate using dual cameras of a smartphone according to an embodiment of the present invention.

At the first step S210, visual data on chest and abdomen movements of the human is collected through the front side camera 160 of the smartphone and PPG signals are collected through the rear side camera 120. At the first step S210, the visual data and the PPG signal are simultaneously measured.

The visual data collected at the first step S210 is a video received from the front side camera 160 of the smartphone 110 with Android OS, and thus it is stored in the YUV420sp signal format. In order to process the visual data, it is necessary to convert the YUV420sp signal format into the RGBA signal format. At the second step S220, the OpenCV library is used to convert the signal format.

At the third step S230, a band-pass filter is applied such that in the converted RGBA signal format, a frequency within a range passes. In order to measure the respiration rate, a low-pass filter ($f_{low}$) of the band-pass filter is set to have a value of 0.08 Hz and a high-pass filter ($f_{high}$) is set to have a value of 2.1 Hz. Also, in order to measure the heart rate, the low-pass filter is set to have a value of 0.667 Hz, and the high-pass filter is set to have a value of 3.833 Hz.

At the fourth step S240, the first and last portions of the RGBA signal to which the band-pass filter is applied are removed which about the first 10 seconds and the last 10 seconds of the signal. This is to remove noise existing in the video signal of the recorded video. Noise occurs at the beginning and the end of collecting the visual data due to actions related to correctly holding the smartphone, a calibration process, etc.

In the present invention, in order to extract optical reflectance from the image signal of the visual data on the chest and abdomen movements, two different region of interest (ROI) images of 49 x 90 pixels are selected from a resolution of 320 x 240 pixels. Also, in order to obtain the PPG pulse signal obtained from the rear side camera 120 of the smartphone 110, a resolution of 176 x 72 pixels is selected from a resolution of 176 x 144 pixels for all frames.

In the re-sized image, the brightness value (I) of each pixel may be obtained by an average value of a RGB channel in the ROI of one image, and Formula is as follows.

Formula 1 [Formula 1]

$$I(x,y,t) = \frac{1}{D}\left(\sum I_b(x,y,t) + \sum I_g(x,y,t) + \sum I_r(x,y,t)\right)$$

Here, D means the size of ROI.

In order to measure the respiration rate, a color channel with the highest standard deviation is selected from three color bands (red, green, and blue) while a green channel is selected to measure the heart rate.

Therefore, at the fifth step S250, the color channel with the highest standard deviation is selected in the RGBA signal with predetermined portions removed at the fourth step.

Next, at the sixth step, a spline filter is applied to the color channel selected at the fifth step. The spline filter is a filter used for smoothing the amplitude of a signal. In the present invention, an average intensity is used to extract an envelope of a filtered discrete signal, and then interpolation and smoothing are performed on the envelope signal.

In the present invention, the sixth step to which the spline filter for smoothing of the signal is applied includes: to obtain a smoothed amplitude envelope signal a(t) in the color channel selected at the fifth step, determining the maximum frequency of the a(t) at the sixth-1 step; obtaining an amplitude time series using cubic spline interpolation at the sixth-2 step; after interpolation, computing a class of an analytic signal of the a(t) at the sixth-3 step; and after determining the maximum value of the a(t), obtaining an average value using sliding window at the sixth-4 step. Cubic spline interpolation at the sixth-2 step is a method used to connect two points that are separated from each other.

At the seventh step, power spectrum density is computed using one of a Welch periodogram, autoregressive power spectrum analysis, and a regression correlation coefficient. The power spectrum is computed using a 512-point fast Fourier transform (FFT) of autocorrelation using a window.

In the present invention, the correlation coefficient for analyzing similarity degree in a time domain between inspiration and expiration is shown in Formula 2.

Formula 2 [Formula 2]

$$corr(x,y) = \frac{cov(x,y)}{\sigma_{x,y}}$$

Here, cov indicates covariance, and $\sigma_x$ and $\sigma_y$ mean standard deviations.

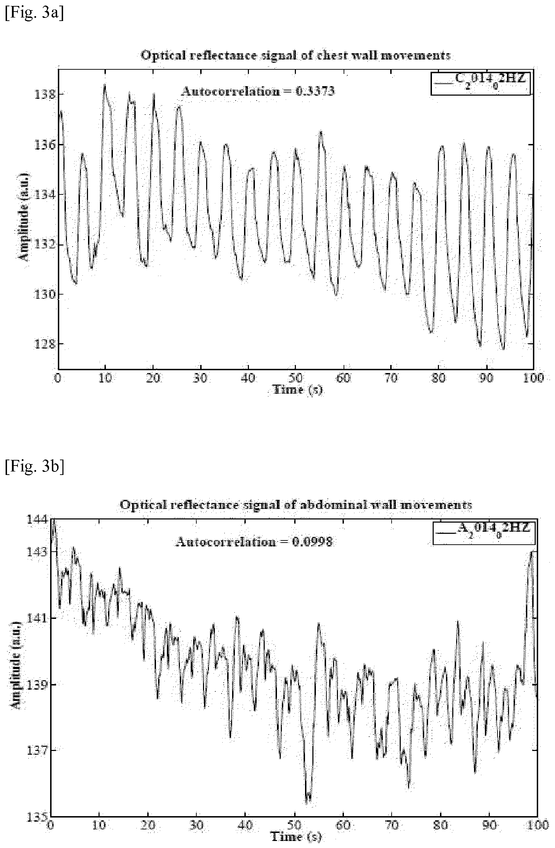

FIGS. 3a and 3b are graphs illustrating respiration rates for the chest and abdomen movements measured using the front side camera 160 of the smartphone 110. Here, correlation coefficient values are 0.3373 and 0.998, respectively. As shown in FIGS. 3a and 3b, since the chest movement is larger than the abdomen movement, a signal quality index is considered to enhance accuracy in measuring the respiration rate. A movement class is assigned to each signal using the correlation coefficient, and the class having a high correlation value is selected. When the correlation coefficient of the chest movement is higher than the correlation coefficient of the abdomen movement, the chest movement is selected.

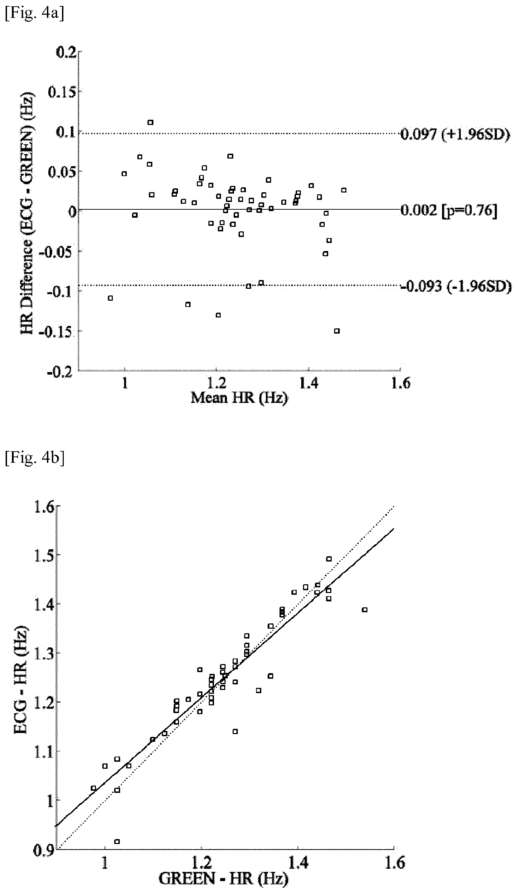

FIG. 4a is a Bland-Altman graph illustrating the heart rate measured through the smartphone. The Bland-Altman graph is a statistical method for comparing two measurement methods, and shows whether there is a difference between the average of two methods and the obtained value or whether there is a difference between an estimated value and an actual measured value. The graph in FIG. 4a shows the differences in measured values between the continuous heart rate measured by the smartphone and corresponding ECG signals. Since there are five points of a scatter plot where z-scores based on standardized rates are outside the confidence interval of 95%, there is little difference between the two methods.

FIG. 4b is a correlation graph of two measurement methods. The x-axis represents continuous heart rate measurement values using the smartphone, and the y-axis represents ECG measurement values. In FIG. 4b, the solid line indicates a regression line and the dotted line indicates Pearson correlation coefficients. Since points of a scatter plot are evenly scattered around the solid line rather spread, a correlation is high.

Last, at the eighth step, the heart rate and the respiration rate are computed using the result of the seventh step.

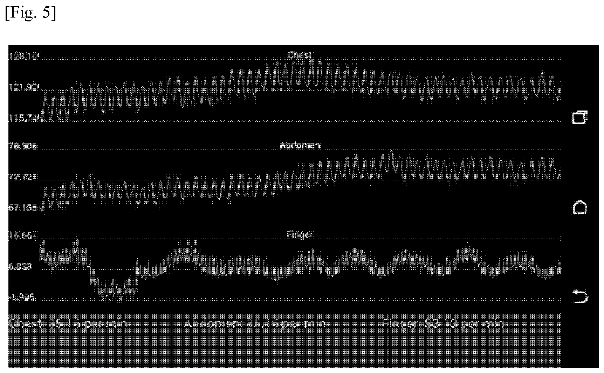

FIG. 5 is a view illustrating a graph of the respiration rate measured for the chest and abdomen movements and the PPG signal of the finger according to an embodiment of the present invention. In the present invention, simultaneously, the respiration rate is measured using the chest and abdomen movements and the PPG signal is measured through the finger, and thus the human can easily observe the respiration rate and the PPG signal in real time.

Accordingly, the present invention is a very useful invention that can be widely applied to a health management system capable of managing heath.

Preferred embodiments of the present invention have been described for illustrative purposes, and should not be construed as being restrictive. The scope of the present invention is defined by the accompanying claims rather than the description which is presented above.

**Claims**

1. A method for simultaneously measuring a respiration rate and a heart rate using dual cameras (120, 160) of a smartphone (110), the method comprising:

   collecting, at a first step (210), a PPG signal of a human with a first camera (120) of a smartphone and visual data on chest and abdomen movements of the human with a second camera (160) of the smartphone;
   converting, at a second step (220), the visual data to an RGBA signal;
   applying, at a third step (230), a bandpass filter such that a frequency only within a range in the RGBA signal passes;
   removing, at a fourth step (240), first and last predetermined time-portions of the RGBA signal to which the band-pass filter is applied at the third step;
   selecting, at a fifth step (250), a color channel having a highest standard deviation from the RGBA signal where the predetermined portions are removed at the fourth step;
   applying, at a sixth step (260), a spline filter to the color channel selected at the fifth step;
   computing, at a seventh step (270), power spectrum density using the result of the sixth step; and
   computing, at an eighth step (280), a heart rate and a respiration rate using the result of the seventh step.

2. The method of claim 1, wherein the PPG signal is measured while a finger of the human is in contact with the first camera (120) provided on a rear side of the smartphone (110).

3. The method of claim 1, wherein the visual data on the chest and abdomen movements of the human is collected using the second camera (160) provided on a front side of the smartphone (110).

4. The method of claim 3, wherein the human wears impedance belts (130, 140) on a chest and an abdomen such that an actual respiration rate is measured.

5. The method of claim 4, wherein at the fourth step, the predetermined time-portions are first 10 seconds and last 10 seconds of the RGBA signal.

6. The method of claim 5, wherein the sixth step comprises:

determining, at a sixth-1 step, in order to obtain a smoothed amplitude envelope signal a(t) in the color channel selected at the fifth step, a maximum frequency of the a(t);
obtaining, at a sixth-2 step, an amplitude time series using cubic spline interpolation;
computing, at a sixth-3 step, a class of an analytic signal of the a(t) after interpolation; and
obtaining, at a sixth-4 step, after determining a maximum value of the a(t), an average value using sliding window.

7. The method of claim 6, wherein at the seventh step, the power spectrum density is computed using one of a Welch periodogram, autoregressive power spectrum analysis, and a regression correlation coefficient.

**Patentansprüche**

1. Ein Verfahren zum gleichzeitigen Messen einer Atemfrequenz und einer Herzfrequenz unter Verwendung von Doppelkameras (120, 160) eines Smartphones (110), wobei das Verfahren folgendes aufweist:

Sammeln, in einem ersten Schritt (210), eines PPG-Signals eines Menschen mit einer ersten Kamera (120) eines Smartphones und visueller Daten über Brust- und Abdomenbewegungen des Menschen mit einer zweiten Kamera (160) des Smartphones;
Umwandeln, in einem zweiten Schritt (220), der visuellen Daten in ein RGBA-Signal;
Anwenden, in einem dritten Schritt (230), eines Bandpassfilters, und zwar derart, dass eine Frequenz nur innerhalb eines Bereichs in dem RGBA-Signal durchgeht;
Entfernen, in einem vierten Schritt (240), von ersten und letzten vorbestimmten Zeitteilen des RGBA-Signals, auf welches der Bandpassfilter in dem dritten Schritt angewendet wird;
Auswählen, in einem fünften Schritt (250), eines Farbkanals mit einer höchsten Standardabweichung von dem RGBA-Signal, wo die vorbestimmten Teile in dem vierten Schritt entfernt werden;
Anwenden, in einem sechsten Schritt (260), eines Spline-Filters auf den im fünften Schritt ausgewählten Farbkanal;
Berechnen, in einem siebten Schritt (270), von Leistungsspektrumsdichte unter Verwendung des Ergebnisses des sechsten Schritts; und
Berechnen, in einem achten Schritt (280), einer Herzfrequenz und einer Atemfrequenz unter Verwendung des Ergebnisses des siebten Schritts.

2. Das Verfahren nach Anspruch 1, wobei das PPG-Signal gemessen wird, während ein Finger des Menschen in Kontakt mit der ersten Kamera (120) ist, welche auf einer Rückseite des Smartphones (110) vorgesehen ist.

3. Das Verfahren nach Anspruch 1, wobei die visuellen Daten zu den Brust- und Abdomenbewegungen des Menschen unter Verwendung der zweiten Kamera (160) gesammelt werden, welche auf einer Vorderseite des Smartphones (110) vorgesehen ist.

4. Das Verfahren nach Anspruch 3, wobei der Mensch Impedanzgurte (130, 140) auf einer Brust und einem Abdomen trägt, und zwar derart, dass eine tatsächliche Atemfrequenz gemessen wird.

5. Das Verfahren nach Anspruch 4, wobei in dem vierten Schritt die vorbestimmten Zeitteile erste 10 Sekunden und letzte 10 Sekunden des RGBA-Signals sind.

6. Das Verfahren nach Anspruch 5, wobei der sechste Schritt folgendes aufweist:

Bestimmen, in einem sechsten-1-Schritt, um ein geglättetes Amplitudenhüllkurvensignal a(t) in dem im fünften Schritt ausgewählten Farbkanal zu erhalten, einer maximalen Frequenz von dem a(t);
Erhalten, in einem sechsten-2-Schritt, einer Amplitudenzeitreihe unter Verwendung einer kubischen Spline-Interpolation;
Berechnen, in einem sechsten-3-Schritt, einer Klasse eines analytischen Signals von dem a(t) nach Interpolation; und

Erhalten, in einem sechsten-4-Schritt, nach Bestimmen eines Maximalwerts von dem a(t), eines Durchschnittswerts unter Verwendung eines gleitenden Fensters.

7. Das Verfahren nach Anspruch 6, wobei in dem siebten Schritt die Leistungsspektrumsdichte unter Verwendung von einem von einem Welch-Periodogramm, einer autoregressiven Leistungsspektrumsanalyse und einem Regressionskorrelationskoeffizienten berechnet wird.

**Revendications**

1. Procédé pour mesurer simultanément la fréquence respiratoire et la fréquence cardiaque, utilisant des caméras doubles (120, 160) d'un mobile multifonction (110), le procédé comprenant :

    la collecte, à une première étape (210), d'un signal PPG d'un humain avec une première caméra (120) d'un mobile multifonction et de données visuelles sur les mouvements de la poitrine et de l'abdomen de l'humain avec une deuxième caméra (160) du mobile multifonction ;
    la conversion, à une deuxième étape (220), des données visuelles en un signal RGBA ;
    l'application, à une troisième étape (230), d'un filtre passe-bande de façon que passe seulement une fréquence dans la plage du signal RGBA ;
    le retrait, à une quatrième étape (240), de première et dernière portions de temps prédéterminées du signal RGBA auquel le filtre passe-bande a été appliqué à la troisième étape ;
    la sélection, à une cinquième étape (250), d'un canal de couleur ayant l'écart-type maximal par rapport au signal RGBA dont les portions prédéterminées ont été retirées à la quatrième étape ;
    l'application, à une sixième étape (260), d'un filtre spline au canal de couleur sélectionné à la cinquième étape ;
    le calcul, à une septième étape (270), de la densité du spectre de puissance par utilisation du résultat de la sixième étape ; et
    le calcul, à une huitième étape (280), de la fréquence cardiaque et de la fréquence respiratoire par utilisation du résultat de la septième étape.

2. Procédé selon la revendication 1, dans lequel le signal PPG est mesuré cependant qu'un doigt de l'humain est en contact avec la première caméra (120) disposée sur le côté arrière du mobile multifonction (110).

3. Procédé selon la revendication 1, dans lequel les données visuelles sur les mouvements de la poitrine et de l'abdomen de l'humain sont collectées par utilisation de la deuxième caméra (160) disposée sur le côté avant du mobile multifonction (110).

4. Procédé selon la revendication 3, dans lequel l'humain porte des sangles d'impédance (130, 140) sur la poitrine et l'abdomen de façon que la fréquence respiratoire réelle soit mesurée.

5. Procédé selon la revendication 4, dans lequel, à la quatrième étape, les portions de temps prédéterminées sont les 10 premières secondes et les 10 dernières secondes du signal RGBA.

6. Procédé selon la revendication 5, dans lequel la sixième étape comprend :

    la détermination, à une sixième-1 étape, afin que soit obtenu un signal d'enveloppe d'amplitude lissé a(t) dans le canal de couleur sélectionné à la cinquième étape, d'une fréquence maximale de l'a(t) ;
    l'obtention, à une sixième-2 étape, d'une série de temps d'amplitude par utilisation d'une interpolation par spline cubique ;
    le calcul, à une sixième-3 étape, d'une classe d'un signal analytique de l'a(t) après l'interpolation ; et
    l'obtention, à une sixième-4 étape, après la détermination de la valeur maximale de l'a(t), d'une valeur moyenne par utilisation d'une fenêtre dynamique.

7. Procédé selon la revendication 6, dans lequel, à la septième étape, la densité du spectre de puissance est calculée par utilisation de l'un parmi un périodogramme de Welch, une analyse de spectre de puissance autorégressive, et un coefficient de corrélation de régression.

[Fig. 1a]

[Fig. 1b]

[Fig. 2]

```
                        ╭─────────────╮
                        │    Start     │
                        ╰──────┬──────╯
                               │            ⌐S210
   ┌───────────────────────────▼───────────────────────────────┐
   │  Collecting PPG signal and visual data on chest and abdomen movements │
   └───────────────────────────┬───────────────────────────────┘
                               │            ⌐S220
   ┌───────────────────────────▼───────────────────────────────┐
   │            Converting visual data to RGBA signal            │
   └───────────────────────────┬───────────────────────────────┘
                               │            ⌐S230
   ┌───────────────────────────▼───────────────────────────────┐
   │               Applying bandpass filter                      │
   └───────────────────────────┬───────────────────────────────┘
                               │            ⌐S240
   ┌───────────────────────────▼───────────────────────────────┐
   │  Removing first and last predetermined portions of RGBA signal │
   │           to which bandpass filter is applied               │
   └───────────────────────────┬───────────────────────────────┘
                               │            ⌐S250
   ┌───────────────────────────▼───────────────────────────────┐
   │  Selecting color channel having highest standard deviation from │
   │     RGBA signal where predetermined portions are removed    │
   └───────────────────────────┬───────────────────────────────┘
                               │            ⌐S260
   ┌───────────────────────────▼───────────────────────────────┐
   │        Applying spline filter to selected color channel     │
   └───────────────────────────┬───────────────────────────────┘
                               │            ⌐S270
   ┌───────────────────────────▼───────────────────────────────┐
   │             Computing power spectrum density                │
   └───────────────────────────┬───────────────────────────────┘
                               │            ⌐S280
   ┌───────────────────────────▼───────────────────────────────┐
   │          Computing heart rate and respiration rate          │
   └───────────────────────────┬───────────────────────────────┘
                               │
                        ╭──────▼──────╮
                        │     End      │
                        ╰─────────────╯
```

[Fig. 3a]

Optical reflectance signal of chest wall movements

Autocorrelation = 0.3373

[Fig. 3b]

Optical reflectance signal of abdominal wall movements

Autocorrelation = 0.0998

[Fig. 4a]

[Fig. 4b]

[Fig. 5]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020120066868
- US 2015124067 A1
- US 2011251493 A1
- KR 1015016903 A
- US 2013215244 A1